# EUROPÄISCHE PATENTSCHRIFT

(11) **EP 3 413 039 B1**
(45) Veröffentlichungstag und Bekanntmachung des Hinweises auf die Patenterteilung: **23.12.2020**
(21) Anmeldenummer: 17174730.6
(22) Anmeldetag: 07.06.2017
(51) Int. Cl.: G01N 25/68

(54) **VORRICHTUNG ZUR BESTIMMUNG DES TAUPUNKTES EINES GASES IN EINEM PROZESSRAUM UND WÄRMEBEHANDLUNGSVORRICHTUNG MIT EINER SOLCHEN VORRICHTUNG ZUR BESTIMMUNG DES TAUPUNKTES**
DEVICE FOR DETERMINING THE DEW POINT OF A GAS IN A PROCESS CHAMBER AND HEAT TREATING APPARATUS HAVING SUCH A DEVICE FOR DETERMINING THE DEW POINT
DISPOSITIF DE DÉTERMINATION DU POINT DE ROSÉE D'UN GAZ DANS UNE CHAMBRE DE PROCESSUS ET DISPOSITIF DE TRAITEMENT THERMIQUE COMPRENANT UN TEL DISPOSITIF DE DÉTERMINATION DU POINT DE ROSÉE

(43) Veröffentlichungstag der Anmeldung: 12.12.2018
(73) Patentinhaber: Belimed AG, 6300 Zug (CH)
(72) Erfinder: Ulhenhaut, Dirk Ingmar, 8032 Zürich (CH); Ganz, Jochen, 8610 Uster (CH)
(74) Vertreter: Hepp Wenger Ryffel AG

(56) Entgegenhaltungen:
- WO-A1-2010/070273
- WO-A1-2011/110197
- CH-A- 438 784
- DE-A1- 1 801 296

## Beschreibung

Die Erfindung betrifft eine Vorrichtung zur Bestimmung des Taupunktes eines Gases in einem Prozessraum und insbesondere zur Bestimmung der Dampfreinheit, bevorzugt der Anwesenheit von nicht kondensierbaren Gasen, in einem Prozessraum. Die Erfindung betrifft ferner eine Wärmebehandlungsvorrichtung mit einer erfindungsgemässen Vorrichtung zur Bestimmung des Taupunktes eines Gases in einem Prozessraum.

Solche Vorrichtungen werden bei der Wärmebehandlung, insbesondere bei der Sterilisation, von Objekten eingesetzt, welche über für das eingesetzte Gas schwer zu erreichende Stellen verfügen, wie das beispielsweise bei Handtuchstapeln oder Enden von Kapillaren oder Endoskopen der Fall ist. Bei der Sterilisierung wird angenommen, dass im gesamten Prozessraum die Gleichgewichtstemperatur zwischen Gas und Flüssigphase erreicht wird. Falls es jedoch, wie oben beschrieben, schwer zu erreichende Stellen vorhanden sind, wird unter Umständen diese Dampfreinheit lokal oder global nicht erreicht. Infolgedessen wird die gewünschte Temperatur nicht oder verzögert erreicht, so dass die Sterilisation als nicht erfolgreich bezeichnet wird.

Es werden daher unterschiedliche Vorrichtungen angewendet, um eine korrekt durchgeführte Sterilisation zu bestätigen. Bekannt sind Sensoren, welche beim Erreichen der erforderlichen Temperatur und Anwesenheit von Feuchtigkeit die Farbe wechseln, und so das Erreichen der erforderlichen Temperatur an der Stelle bestätigen. Nachteilig ist jedoch, dass eine Kontrolle erst nach dem gesamten Prozessverlauf möglich ist. Ferner sind Farbumschlagsensoren hinsichtlich der Genauigkeit und der Zuverlässigkeit ebenfalls mit Nachteilen behaftet, da der Farbumschlag nicht nur von der Temperatur sondern auch von der Feuchtigkeit und der Einwirkzeit abhängig ist.

Andere Vorrichtungen sehen die Anordnung eines wärmeleitenden Körpers in einem Prozessraum vor. Der wärmeleitende Körper ist mit einer Temperiervorrichtung zum Ändern der Temperatur des Körpers verbunden und mit Temperatursensoren versehen. Durch Erwärmung und Abkühlung des Körpers wird eine Kondensation des Prozessgases am Körper selber erzeugt, wobei die freigesetzte Kondensationswärme eine Unstetigkeit der Abkühlungskurve des Körpers verursacht. Eine solche Vorrichtung ist aus der EP 0 286 834 bekannt. Die Querschnittsfläche des Körpers ist kleiner als die betaubare Oberfläche des Körpers, so dass die Temperatur der betaubaren Oberfläche durch die Temperatur des Kondensats bestimmt wird. Nachteilig ist bei einer solchen Anordnung, dass diese nicht beliebig miniaturisiert werden kann, weil die betaubare Oberfläche stets ein gewisses Verhältnis zur Querschnittsfläche aufweisen muss. Zudem muss die Vorrichtung teilweise ausserhalb des Prozessraumes angeordnet werden, was das Einsatzspektrum einer solchen Vorrichtung beschränkt.

DE1801296 A1 offenbart eine Vorrichtung zum Messen des Taupunkts aufweisend einen thermoelektrischen Kühler und ein Thermoelement, das in direktem Kontakt mit dem thermoelektrischen Kühler ist.

Es ist daher Aufgabe der Erfindung eine Vorrichtung zur Bestimmung des Taupunktes eines Gases anzugeben, welche die Nachteile des Bekannten vermeidet und insbesondere eine Überwachung quasi in Echtzeit des Taupunktes eines Gases ermöglicht und gleichzeitig miniaturisierbar ist.

Die Aufgabe wird mit einer Vorrichtung nach dem unabhängigen Anspruch gelöst. Die erfindungsgemässe Vorrichtung zur Bestimmung des Taupunktes eines Gases in einem Prozessraum umfasst ein Temperierelement und einen Temperatursensor zur Bestimmung der Temperatur im Prozessraum, welcher in wärmeleitender Wirkverbindung mit dem Temperierelement steht. Das Temperierelement ist zum aktiven Erwärmen und Abkühlen des Temperatursensors ausgebildet.

Erfindungsgemäss ist der Temperatursensor in direktem Kontakt am Temperierelement angeordnet.

Damit kann auf den bei den bekannten Vorrichtungen verwendeten wärmeleitenden Körper verzichtet werden. Der Temperatursensor selbst dient auch als wärmeleitender Körper. Damit ist eine Miniaturisierung der Vorrichtung möglich. Zudem ist die thermische Masse des Temperatursensors sehr klein, so dass die Temperatur des Temperatursensors durch ein kleines Temperierelement geändert werden kann. Da der Temperatursensor direkt am Temperierelement angeordnet ist, entfällt ferner auch die Notwendigkeit eines zweiten Temperatursensors, da die Temperatur des Temperierelements der Temperatur des Temperatursensors aufgrund der Anordnung am Temperierelement und der kurzen Wärmeleitwege entspricht. Es ist ersichtlich, dass der Temperatursensor die Temperatur im Prozessraum nur bestimmen kann, wenn das Temperierelement nicht betrieben wird. Sobald das Temperierelement betrieben wird und den Temperatursensor aufheizt oder abkühlt, wird vom Temperatursensor seine eigene Temperatur bestimmt.

Die erfindungsgemässe Vorrichtung wird wie folgt betrieben: Zunächst wird die Temperatur des Temperierelements erhöht, so dass auch die Temperatur des Temperatursensors über die Kondensationstemperatur des Gases bei den im Prozessraum herrschenden Bedingungen erhöht wird. Damit wird sichergestellt, dass die Messfläche des Temperatursensors, welche gleichzeitig als betaubare Oberfläche dient, trocken ist. Das Temperierelement wird dann abgekühlt, so dass die Kondensation an der Messfläche des Temperatursensors ermöglicht wird. Bei der Kondensation des Gases wird Kondensationswärme freigesetzt, welche die Abkühlungsrate des Temperatursensors beeinflusst. Dies ist als Unstetigkeit (z.B. als "Knick") in einer graphischen Darstellung des Temperaturverlaufes des Temperatursensors ersichtlich. Im besten Fall entsteht ein Gleichgewicht, so dass in der graphischen Darstellung eine Plateauphase bei der Taupunktemperatur erkennbar ist. Damit ist der Taupunkt des Gases in der unmittelbaren Umgebung der Vorrichtung messbar.

Bei einer bevorzugten Ausführungsform sind der Temperatursensor und das Temperierelement voneinander elektrisch isoliert.

Eine solche Anordnung ist insbesondere notwendig, wenn das Temperierelement aus einem elektrisch leitenden Material besteht und der Temperatursensor ebenfalls elektrisch leitende Teile aufweist (z.B. Drähte), welche direkt am Temperierelement angeordnet sind und sonst zu einem Kurzschluss bzw. den Betrieb der Vorrichtung beeinträchtigen könnten. Es versteht sich, dass die elektrische Isolierung weiterhin eine gute Wärmeleitung zwischen Temperatursensor und Temperierelement erlaubt.

Dabei ist zu vermerken, dass im Sinne der vorliegenden Erfindung, auch wenn zwischen Temperierelement und Temperatursensor eine elektrisch isolierende Schicht vorhanden ist, der Temperatursensor trotzdem als in direktem Kontakt mit dem Temperierelement verstanden wird, da ein wärmeleitender Körper (Heatpipe) dazwischengeschaltet ist.

Bevorzugt ist das Temperierelement als Peltier-Element ausgebildet.

Peltier-Elemente zeichnen sich insbesondere durch eine sehr schnelle Ansprechzeit bei der Änderung der Temperatur aus und können ebenfalls schnell Wärme einem Körper (in diesem Fall dem Temperatursensor) zu- und abführen. Zudem können die Peltier-Elemente sehr klein ausgeführt werden, so dass die Vorrichtung insgesamt sehr klein ausführbar ist.

Bevorzugt ist das Temperierelement mit einem Kühlkörper versehen.

Somit kann die Wärme, welche beim Abkühlen des Temperatursensors demselben entzogen wird, besser abgeführt werden. Insbesondere ist ein Kühlkörper bei einem als Peltier-Element ausgebildeten Temperierelement vorteilhaft. Der Kühlkörper wird an der Seite des Peltier-Elements angeordnet, welche beim Abkühlen des Temperatursensors erwärmt wird, um eine bessere Abkühlung des Temperatursensors zu gewährleisten.

Gemäß der Erfindung ist der Temperatursensor als Thermoelement ausgebildet. Ein Thermoelement besteht im Wesentlichen aus zwei Leitern aus unterschiedlichen Materialien, welche an einem Ende miteinander verbunden sind.

Die Verbindungsstelle eines ersten Leiters und eines zweiten Leiters des Thermoelements bildet dabei eine betaubare Oberfläche. Die Verbindungsstelle, welche in der Regel als Schweisspunkt beider Leitungen ausgebildet ist, stellt erfindungsgemäß die einzige betaubare Oberfläche der Vorrichtung bereit, welche im bestimmungsgemässen Zustand der Vorrichtung im Prozessraum angeordnet wird und zur Bestimmung des Taupunktes Anwendung findet.

Die Verbindungsstelle kann dabei ebenfalls am Temperierelement angeordnet oder von diesem leicht beabstandet sein. Wichtig ist für die vorliegende Erfindung, dass die Verbindungsstelle in wärmeleitender Wirkverbindung mit dem Temperierelement steht und dass der Abstand zwischen Verbindungsstelle und Temperierelement möglichst klein gehalten wird, um die Einflüsse der thermischen Wärmeleitung gering zu halten.

Bevorzugt sind das Thermoelement, mit Ausnahme der Verbindungsstelle beider Leiter, und zumindest Teile des Temperierelements von einer wärmeisolierenden und gasundurchlässigen Schicht umgeben.

Somit wird erreicht, dass ein Wärmeaustausch mit der Umgebung (Prozessraum) nur über die nicht geschützte Stelle der Vorrichtung erfolgt (Verbindungsstelle), so dass die Messwerte nicht von Wärmeaustauschprozessen der übrigen Vorrichtung beeinflusst werden. Zudem schützt die Schicht die übrige Vorrichtung vor Feuchte und Korrosion. Teile des Temperierelements, insbesondere wenn ein Kühlkörper vorhanden ist, werden nicht durch die Schicht geschützt, um eine möglichst grosse Wärmeabfuhr beim Abkühlen des Temperierelements zu erlauben.

Als Materialen für die Schicht finden insbesondere Duroplaste, Lacke, Gasphasenabscheidungen und besonders bevorzugt synthetische Harze Anwendung.

Wie vorher beschrieben stellt die Verbindungsstelle beider Leitungen des Thermoelements die einzige betaubare Oberfläche der Vorrichtung bereit. Es kann jedoch der Fall sein, dass die Schicht im Übergangsbereich zur Verbindungsstelle aufgrund der geringen Schichtdicke der Schicht ebenfalls teilweise beim Betreiben der Vorrichtung betaut wird. Diese Nebeneffekte können bei der genauen Auswahl von Material und Ausführung der Schicht jedoch weitestgehend vernachlässigt werden.

Die Verbindungsstelle ist bevorzugt mit einer wärmeleitenden und gasundurchlässigen Schicht beschichtet.

Eine solche Beschichtung kann bei Ausführungsformen mit und ohne wärmeisolierende und gasundurchlässige Schicht verwendet werden und schützt die Verbindungsstelle, insbesondere vor Korrosion.

Bei einer alternativen Ausführungsform der Vorrichtung, die nicht gemäß der Erfindung ist, ist der Temperatursensor als resistiver Temperatursensor ausgebildet. Insbesondere ist der Temperatursensor als Platin-Messwiderstand ausgebildet, welcher besonders bevorzugt auf das Temperierelement aufgedampft wird.

Eine solche Vorrichtung ist besonders einfach in der Herstellung und erlaubt, je nach Einsatzzweck, die Bereitstellung einer grösseren betaubaren Oberfläche als bei Vorrichtungen mit einem Thermoelement.

Diese Variante eignet sich insbesondere bei der Bestimmung des Taupunktes bei Prozessraumvolumina, welche im Verhältnis zur betaubaren Oberfläche quasi als unendlich angesehen werden können. Der Temperatursensor ist bevorzugt von einer wärmeleitenden und gasundurchlässigen Schutzschicht bedeckt, welche eine betaubare Oberfläche bildet. Die Schutzschicht stellt somit die einzige betaubare Oberfläche der Vorrichtung bereit, welche im bestimmungsgemässen Zustand der Vorrichtung im Prozessraum angeordnet wird und zur Bestimmung des Taupunktes Anwendung findet.

Die Schutzschicht schützt den Sensor vor Feuchte und Korrosion und erlaubt gleichzeitig eine Messung der Temperatur, da die Schutzschicht wärmeleitend ausgebildet ist.

Bevorzugt umfasst die Vorrichtung ferner einen zweiten am Temperierelement angeordneten Temperatursensor zur Ermittlung der Temperatur des Temperierelements.

Eine zweite Temperaturmessung wird bevorzugt zu Verstärkung der Messsignale verwendet. Statt des Signals aus dem ersten Temperatursensor (Temperatur) wird zur Bestimmung des Taupunktes eine Differenz zwischen der vom ersten Temperatursensor gemessenen Temperatur, welche vom Kondensationsvorgang an der betaubaren Oberfläche beeinflusst wird, und der vom zweiten Temperatursensor gemessenen Temperatur, welche im Wesentlichen der Temperatur des Temperierelements entspricht, verwendet. Beide Temperaturen haben beim Abkühlen des Temperatursensors, wenn keine Kondensation an der betaubaren Oberfläche stattfindet, ähnliche Verläufe (die Steigung ist im Wesentlichen gleich), so dass eine Differenz beider Temperaturen in etwa konstant ist. Dies ändert sich sobald die Kondensation an der betaubaren Oberfläche ihren Gang nimmt, da die zweite Temperatur schneller abnimmt als die erste Temperatur (idealerweise bleibt sogar die erste Temperatur konstant). Der Differenzwert nimmt somit zu. Die Zunahme des Differenzwertes wird demnach zur Bestimmung des Taupunktes herangezogen.

Die Vorrichtung umfasst ferner bevorzugt einen Drucksensor zur Ermittlung des Druckes im Prozessraum.

In der Regel ist der im Prozessraum herrschende Druck bekannt. Der gemessene Taupunkt kann somit mit einem Referenzwert aus einer Sattdampftabelle verglichen werden, um festzustellen, ob die gemessenen Bedingungen im Prozessraum tatsächlich den erwarteten und für die Sterilisation notwendigen Bedingungen entsprechen. Mit der Integrierung eines Drucksensors kann eine Vorrichtung bereitgestellt werden, welche eine umfassende Überwachung des

Prozessraumes erlaubt. Durch Heranziehen des Druckes kann die Prozesssicherheit erhöht werden, da einerseits Druckabweichungen im Prozessraum erkannt werden können (z.B. in einem Hohlraum wo der Druck mit einer Verzögerung gegenüber dem übrigen Prozessraum eingestellt wird), andererseits eine Redundanz der Drucksensoren vorhanden ist.

Die Vorrichtung umfasst ferner eine Steuer- und Auswerteeinheit, welche dazu ausgebildet ist, die Taupunkttemperatur anhand von Unstetigkeiten im Temperaturverlauf der von dem Temperatursensor ermittelten Temperatur zu bestimmen.

Die Steuer- und Auswerteeinheit kann mit der Vorrichtung integral ausgebildet sein oder über eine Drahtverbindung oder drahtlose Verbindung mit der Vorrichtung selbst in Kommunikationsverbindung stehen. Wie bereits eingangs erwähnt, wird beim Abkühlen des Temperatursensors, bevorzugt mit einer konstanten Abkühlrate, und beim Erreichen der Taupunkttemperatur an der betaubaren Oberfläche des Temperatursensors die Kondensationswärme des Gases im Prozessraum freigesetzt. Dies führt zu einer Unstetigkeit des Temperaturverlaufes, welche in einer graphischen Darstellung beispielsweise als "Knick" der Abkühlungskurve wiedergeben wird. Die Temperatur, bei welcher die Unstetigkeit auftritt, wird zur Bestimmung der Taupunkttemperatur bei der in der Umgebung des Temperatursensors verwendet. Je nach Abkühlungsrate des Temperatursensors wird das Gas mehr oder weniger unterkühlt, so dass die Temperatur, bei welcher die Unstetigkeit auftritt, nicht exakt der Taupunkttemperatur entspricht, so dass eine Korrektur, welche jedoch Vorrichtungs- und Verfahrensspezifisch ermittelbar ist, unter Umständen notwendig ist.

Die Erfindung betrifft ferner eine Wärmebehandlungsvorrichtung umfassend einen Prozessraum, welcher mit einem Prozessgas beschickbar ist, und wenigstes eine Vorrichtung zur Bestimmung des Taupunktes wie oben beschrieben. Bei der Wärmebehandlungsvorrichtung handelt es sich bevorzugt um eine Sterilisationsvorrichtung, bei welcher den Prozessraum mit Dampf beschickt wird.

Mit der Integration einer erfindungsgemässen Vorrichtung zur Bestimmung des Taupunktes in einer Wärmebehandlungsvorrichtung kann eine erhöhte Prozesssicherheit erreicht werden, wobei die quasi in Echtzeit stattfindende Überwachung des Taupunktes beispielsweise den Abbruch einer Behandlung erlaubt, wenn die Bedingungen für eine erfolgreiche Wärmebehandlung nicht erfüllt werden. Somit sind auch Kosten- und Energieeinsparungen möglich, da nicht zuerst das Ende der Wärmebehandlung erreicht werden muss, um festzustellen, ob die Wärmebehandlung selbst erfolgreich war.

Abschliessend sei noch angemerkt, dass in der obenstehenden Beschreibung stets die Rede von einem Prozessraum bzw. von der Bestimmung des Taupunktes eines Gases im Prozessraum die Rede ist. Es ist jedoch ersichtlich, dass unter Umständen der Prozessraum, in welchem der Taupunkt bestimmt wird (z.B. Innenraum eines Endoskops), nicht mit einem Gesamtprozessraum (z.B. Behandlungskammer eines Sterilisators) übereinstimmt. Anders ausgedrückt kann der Prozessraum, in welchem der Taupunkt bestimmt wird, eine Untereinheit eines grösseren, zweiten Prozessraumes sein.

Die Erfindung wird nachfolgend anhand von bevorzugten Ausführungsbeispielen in Verbindung mit den Figuren besser beschrieben. Es zeigen:
- Fig. 1: Eine schematische Schnittansicht einer Sterilisationsvorrichtung mit einem eingelegten Objekt;
- Fig. 2: eine schematische, perspektivische Ansicht einer ersten Ausführungsform der Vorrichtung;
- Fig. 3: eine Schnittansicht durch die Vorrichtung der Figur 2;
- Fig. 4: eine schematische, perspektivische Ansicht einer zweiten, nicht erfindungsgemäßen, Ausführungsform der Vorrichtung; und
- Fig. 5: eine Schnittansicht durch die Vorrichtung der Figur 4.

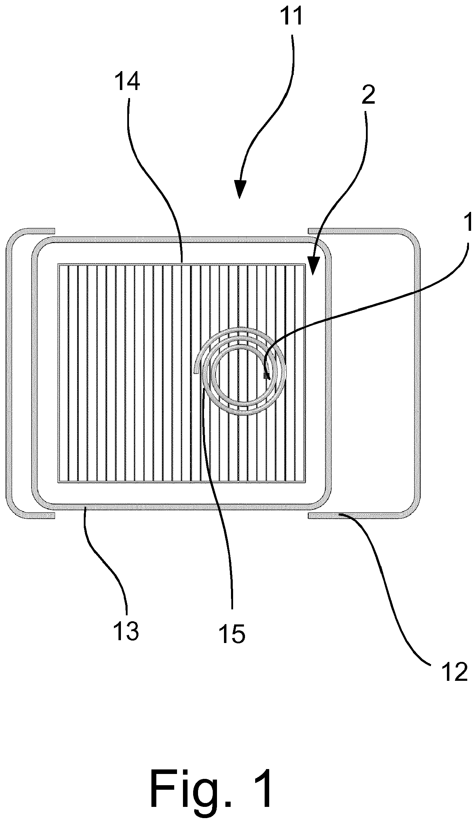

In der Figur 1 ist schematisch eine Wärmebehandlungsvorrichtung 11 dargestellt. Die Wärmebehandlungsvorrichtung 11 umfasst ein Gehäuse 12 mit einer Kammerwand 13, welche einen Prozessraum 2 definiert. Der Prozessraum, welcher hermetisch abgeschlossen ist, wird beim Sterilisieren mit einer Sattdampfatmosphäre beschickt und für eine festgelegte Zeit so gehalten, bis sichergestellt werden kann, dass die für eine Sterilisation notwendige Abtötung von Krankheitserregern stattgefunden hat.

Eine Beladungsträgeretage 14 in Form eines Gitters ist im Prozessraum 2 angeordnet. Auf der Beladungsträgeretage 14 ist schematisch eine aufgerollte Endoskopattrappe 15 gezeigt.

Ein Endoskop ist meistens als schlauchförmiger Hohlkörper ausgebildet. Aus diesem Grund kann nicht sichergestellt werden, dass auch im Hohlraum des Endoskops sich die gleiche Sattdampfatmosphäre wie im übrigen Prozessraum 2 eingestellt hat. Um eine Kontrolle der im Hohlraum des Endoskops herrschenden Bedingungen zu ermöglichen wird somit eine erfindungsgemässe Vorrichtung 1 angeordnet, welche nachfolgend besser beschrieben wird.

Ein Endoskop wird bei der nachfolgenden Beschreibung als rein beispielhaftes Objekt herangezogen. Die erfindungsgemässe Vorrichtung ist jedoch überall einsetzbar, wo die Bedingungen in einem Teilbereich einer Wärmebehandlungsvorrichtung von den Bedingungen im übrigen Prozessraum 2 abweichen können, wie das zum Beispiel bei Handtuchstapeln, Kapillaren, usw. der Fall ist.

An dieser Stelle sei vermerkt, dass in der Regel die Vorrichtung 1 nicht an einem "echten" Endoskop angeordnet wird sondern an einer Endoskopattrappe, welche die gleichen Eigenschaften wie das zu sterilisierende Endoskop 15 aufweist (Durchmesser, Material usw.), da es bei "echten" Endoskopen oft nicht möglich ist, eine Vorrichtung 1 überhaupt anzuschliessen. Da man davon ausgeht, dass in der Mitte eines "echten" Endoskopes die Wahrscheinlichkeit einer Abweichung von den Bedingungen im Prozessraum 2 am höchsten ist, wird die Endoskopattrappe 15 mit der halben Länge des "echten" Endoskops und mit einem geschlossenem Ende ausgeführt. Die Vorrichtung 1 wird an dem geschlossenen Ende der Endoskopattrappe 15 angeordnet.

Es ist ferner zu vermerken, dass in der nachfolgenden Beschreibung von einem Prozessraum 2 und von einem Hohlraum eines Endoskops die Rede ist. Der Hohlraum des Endoskops ist Teil des Prozessraumes 2. Aufgrund der Eigenschaften des Endoskops kann es jedoch der Fall sein, dass im Hohlraum des Endoskops und im übrigen Prozessraum 2 nicht die gleichen Bedingungen herrschen.

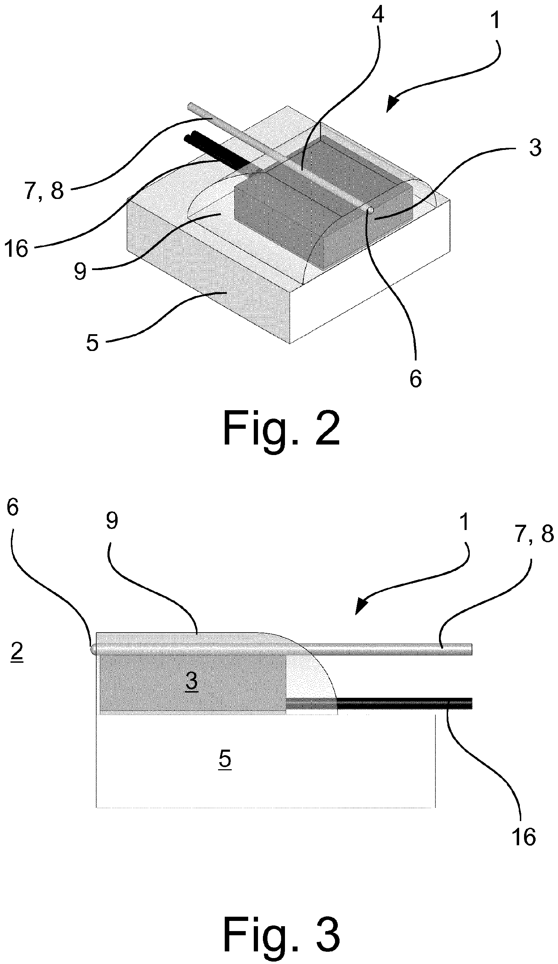

In den Figuren 2 und 3 ist eine erste Ausführungsform der Vorrichtung 1 gezeigt.

Die Vorrichtung 1 umfasst ein Peltier-Element 3, welches auf einem Aluminiumkühlelement 5 angeordnet ist. Das Aluminiumkühlelement 5 kann Kühlrippen aufweisen, welche jedoch der Einfachheit halber in den Figuren 2 bis 5 nicht dargestellt sind. Ferner ist eine Einspeisung 16 für den Betrieb des Peltier-Elements 3 vorhanden.

Auf dem Peltier-Element 3 ist ein Thermoelement 4 angeordnet. Das Thermoelement 4 umfasst zwei Leiter 7 und 8, welche bei einer Verbindungsstelle 6 miteinander verbunden sind. Wie aus der Figur 2 ersichtlich, ist die Verbindungsstelle 6 im bestimmungsgemässen Zustand dem Prozessraum 2 (oder besser gesagt dem Hohlraum des Endoskops 15) zugewandt.

Das Thermoelement 4 ist in wärmeleitender Wirkverbindung mit dem Peltier-Element 3, jedoch von Letzterem mittels einer nicht dargestellten Schicht elektrisch isoliert. Das Thermoelement 4 kann somit mittels des Peltier-Elements 3 geheizt und abgekühlt werden.

Eine der Übersichtlichkeit halber als durchsichtig dargestellte Kunstharzmasse 9 umgibt das Peltier-Element 3 und das Thermoelement 4 und schützt diese vor Feuchtigkeit und Korrosion. Gleichzeitig dient die Kunstharzmasse 9 als thermische Isolierung für die Vorrichtung 1, so dass ein Wärmeaustausch mit dem Hohlraum der Endoskopattrappe 15 nur über die nicht geschützten Bereiche der Vorrichtung 1 stattfinden kann. Das Kühlelement 5 ist nur teilweise von der Kunstharzmasse 9 umgeben, da die vom Peltier-Element 3 an der Kontaktfläche zwischen Peltier-Element 3 und Kühlelement 5 beim Abkühlen des Thermoelements 4 erzeugte Wärme abgeführt werden soll. Je nach Anwendung können weitere Teile des Kühlelements 5 von der Kunstharzmasse 9 umgeben sein. So kann es beispielsweise der Fall sein, dass das Kühlelement 5 die Wärme im Prozessraum 2 ausserhalb des Hohlraumes der Endoskopattrappe 15 abgibt, um die Atmosphäre im Hohlraum nicht zu beeinflussen.

Lediglich die Verbindungsstelle 6 ragt aus der Kunstharzmasse 9 heraus und in den Prozessraum 2 hinein und erlaubt somit die Bestimmung des Taupunktes, welche später beschrieben wird. Es ist ferner ersichtlich, dass ebenfalls die Leitungen 7 und 8 des Thermoelements 4 sowie die Einspeisung 16 des Peltier-Elements 3 nur teilweise von der Kunstharzmasse 9 geschützt werden. Dies hat keinen Einfluss auf die Vorrichtung 1, da die Leitungen 7 und 8 sowie die Einspeisung 16 ebenfalls von einer Isolierung umgeben sind.

Zur Bestimmung des Taupunktes wird das Thermoelement 4 zunächst mithilfe des Peltier-Elements auf eine Temperatur oberhalb des Taupunktes des sich im Prozessraum 2 befindlichen Gases aufgewärmt, um sicherzustellen, dass kein kondensiertes Gas sich auf der Oberfläche der Verbindungsstelle 6 befindet. Das Thermoelement 4 wird anschliessend mit einer konstanten Abkühlrate abgekühlt. Beim Erreichen der Taupunkttemperatur findet eine Kondensation des Gases an der Oberfläche der Verbindungsstelle 6 statt. Die dadurch freigesetzte Kondensationswärme beeinflusst die Abkühlung des Thermoelements 6, so dass eine Unstetigkeit des Temperaturverlaufes entsteht. Der Taupunkt kann somit sehr einfach durch Bestimmung der Unstetigkeit im Temperaturverlauf ermittelt werden.

Beispielsweise kann eine erfindungsgemässe Vorrichtung 1 zunächst für 2 bis 5 Sekunden über die Taupunkttemperatur erwärmt werden und anschliessend mit einer Abkühlrate von 10 K/s abgekühlt werden. Der Taupunkt kann somit in weniger als 10 Sekunden ermittelt werden. Dies erlaubt durch Wiederholung der Taupunktbestimmung eine kontinuierliche Überwachung der stattfindenden Wärmebehandlung, so dass diese, falls die notwendigen Bedingungen im Hohlraum nicht erfüllt werden, sofort gestoppt und neu gestartet werden kann. Damit kann die Prozesssicherheit erhöht werden, da quasi in Echtzeit überwacht werden kann, ob die Wärmebehandlung erfolgreich war.

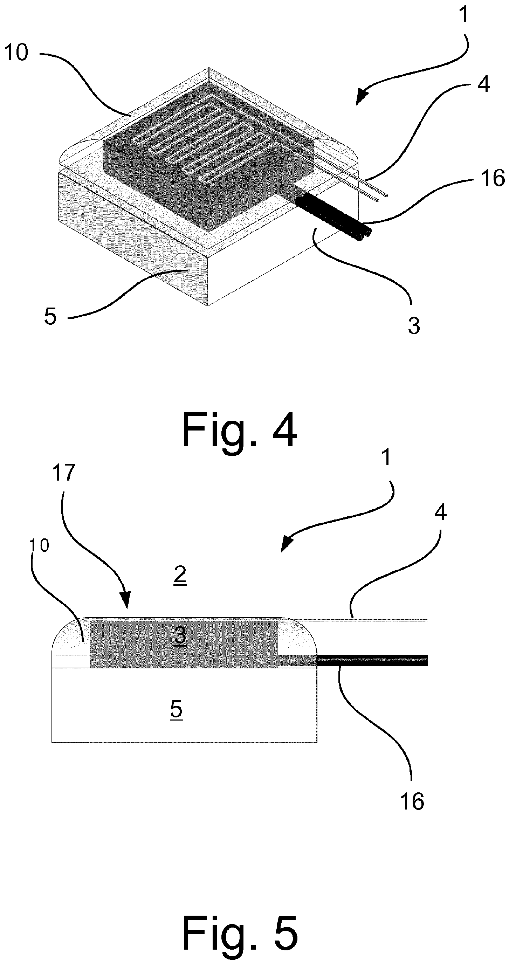

In den Figuren 4 und 5 ist eine zweite, nicht erfindungsgemäße, Ausführungsform der Vorrichtung 1 gezeigt.

Die Vorrichtung 1 umfasst ein Peltier-Element 3, welches auf einem Aluminiumkühlelement 5 angeordnet ist. Ferner ist eine Einspeisung 16 für den Betrieb des Peltier-Elements 3 vorhanden. Auf dem Peltier-Element 3 ist ein resistiver Temperatursensor 4 angeordnet. Der mäanderförmige Verlauf des Temperatursensors 4 erhöht die Länge der resistiven Strecke des Temperatursensors 4, so dass eine präzisere Messung der Temperatur möglich ist bzw. stärkere Messwerte erzeugt werden. Der Temperatursensor 4 kann beispielsweise als aufgedampfter Platindraht ausgebildet sein. Der Temperatursensor 4 ist in wärmeleitender Wirkverbindung mit dem Peltier-Element 3, jedoch von Letzterem mittels einer nicht dargestellten Schicht elektrisch isoliert. Der Temperatursensor 4 kann somit mittels des Peltier-Elements 3 geheizt und abgekühlt werden.

Wie aus der Figur 5 ersichtlich, ist der Temperatursensor 4 im bestimmungsgemässen Zustand auf der Seite des Peltier-Elements 3 angeordnet, welche dem Prozessraum 2 (oder besser gesagt dem Hohlraum der Endoskopattrappe 15) zugewandt ist.

Eine der Übersichtlichkeit halber als durchsichtig dargestellte Schutzschicht 10 umgibt das Peltier-Element 3 und schützt dieses vor Feuchtigkeit und Korrosion. Gleichzeitig dient die Schutzschicht 10 als thermische Isolierung für die Vorrichtung 1, so dass ein Wärmeaustausch mit dem Prozessraum 2 nur über den Bereich 17, in welchem der Temperatursensor 4 freiliegt, erfolgen kann.

Das Kühlelement 5 ist ferner nur teilweise von der Schutzschicht 10 umgeben, da die vom Peltier-Element 3 an der Kontaktfläche zwischen Peltier-Element 3 und Kühlelement 5 beim Abkühlen des Temperatursensors 4 erzeugte Wärme abgeführt werden soll. Je nach Anwendung können weitere Teile des Kühlelements 5 von der Schutzschicht 10 umgeben sein. So kann beispielsweise der Fall sein, dass das Kühlelement 5 die Wärme im Prozessraum 2 aber ausserhalb des Hohlraumes der Endoskopattrappe 15 abgibt, um die Atmosphäre im Hohlraum nicht zu beeinflussen.

Die Messung des Taupunktes erfolgt analog zur Messung mit einer Vorrichtung 1 gemäss den Figuren 2 und 3 mit dem Unterschied, dass die betaubare Oberfläche die Oberfläche des Temperatursensors 4 ist. Diese betaubare Oberfläche wird zunächst erwärmt und anschliessend abgekühlt, bis die Kondensation des Gases einsetzt. Auch in diesem Fall führt die freigesetzte Kondensationswärme dazu, dass eine Unstetigkeit im Temperaturverlauf bei der Abkühlung der betaubaren Oberfläche auftritt.

## Patentansprüche

1. Vorrichtung (1) eingerichtet zur Bestimmung des Taupunktes eines Gases in einem Prozessraum (2) umfassend:
- ein Temperierelement (3) und
- einen Temperatursensor (4) zur Bestimmung der Temperatur einer betaubaren Messfläche des Temperatursensors (3), welcher in wärmeleitender Wirkverbindung mit dem Temperierelement (3) steht,
wobei das Temperierelement (3) zum aktiven Erwärmen und Abkühlen des Temperatursensors (4) ausgebildet ist, wobei der Temperatursensor in Kontakt am Temperierelement (3) angeordnet ist, und wobei der Temperatursensor (4) als Thermoelement ausgebildet ist, **dadurch gekennzeichnet, dass**
die Vorrichtung (1) dazu eingerichtet ist zur Bestimmung des Taupunktes des Gases im Prozessraum (2) die Verbindungsstelle (6) eines ersten Leiters (7) und eines zweiten Leiters (8) des Thermoelements (4) als einzige Oberfläche der Vorrichtung (1) zu betauen.

2. Vorrichtung (1) nach Anspruch 1, **dadurch gekennzeichnet, dass** der Temperatursensor (4) und das Temperierelement (3) voneinander elektrisch isoliert sind.

3. Vorrichtung (1) nach Anspruch 1 oder 2, **dadurch gekennzeichnet, dass** das Temperierelement (3) als Peltier-Element ausgebildet ist.

4. Vorrichtung (1) nach einem der vorhergehenden Ansprüche, **dadurch gekennzeichnet, dass** das Temperierelement (3) mit einem Kühlkörper (5) versehen ist.

5. Vorrichtung (1) nach Anspruch 1, **dadurch gekennzeichnet, dass** eine wärmeisolierende und gasundurchlässige Schicht (9) das Thermoelement (4), mit Ausnahme der Verbindungsstelle (6) beider Leiter (7, 8), und bevorzugt zumindest Teile des Temperierelements (4) umgibt.

6. Vorrichtung nach einem der Ansprüche 1 oder 5, **dadurch gekennzeichnet, dass** die Verbindungsstelle (6) mit einer wärmeleitenden und gasundurchlässigen Schicht überzogen ist.

7. Vorrichtung (1) nach einem der vorhergehenden Ansprüche, **dadurch gekennzeichnet, dass** die Vorrichtung (1) ferner einen zweiten am Temperierelement angeordneten Temperatursensor zur Ermittlung der Temperatur des Temperierelements (4) umfasst.

8. Vorrichtung (1) nach einem der vorhergehenden Ansprüche, **dadurch gekennzeichnet, dass** die Vorrichtung (1) ferner einen Drucksensor zur Ermittlung des Druckes im Prozessraum (2) umfasst.

9. Vorrichtung (1) nach einem der vorhergehenden Ansprüche, **dadurch gekennzeichnet, dass** die Vorrichtung (1) ferner eine Steuer- und Auswerteeinheit umfasst, welche dazu ausgebildet ist, die Taupunkttemperatur anhand von Unstetigkeiten im Temperaturverlauf der von dem Temperatursensor (4) ermittelten Temperatur zu bestimmen.

10. Wärmebehandlungsvorrichtung (11), insbesondere Sterilisationsvorrichtung umfassend einen Prozessraum (2), welcher mit einem Prozessgas beschickbar ist, und wenigstes eine Vorrichtung (1) zur Bestimmung des Taupunktes nach einem der vorhergehenden Ansprüche.

## Claims

1. Device (1) arranged for determining the dew point of a gas in a process chamber (2) comprising:
- a temperature control element (3) and
- a temperature sensor (4) for determining the temperature of a dewable measuring surface of the temperature sensor (3), which is in thermally conductive active connection with the temperature control element (3),
wherein the temperature control element (3) is designed for actively heating and cooling the temperature sensor (4),
wherein
the temperature sensor is arranged in contact on the temperature control element (3), and wherein
the temperature sensor (4) is designed as a thermocouple, **characterised in that**
the device (1) is designed to determine the dew point of the gas in the process chamber (2) by dewing the junction (6) of a first conductor (7) and a second conductor (8) of the thermocouple (4) as the only surface of the device (1).

2. Device (1) according to claim 1, **characterised in that** the temperature sensor (4) and the temperature control element (3) are electrically insulated from each other.

3. The device (1) according to claim 1 or 2, **characterised in that** the temperature control element (3) is designed as a Peltier element.

4. The device (1) according to one of the preceding claims, **characterised in that** the temperature control element (3) is provided with a heat sink (5).

5. The device (1) according to claim 1, **characterised in that** a heat-insulating and gas-impermeable layer (9) surrounds the thermocouple (4), with the exception of the junction (6) of the two conductors (7, 8), and preferably at least parts of the temperature control element (4).

6. The device according to one of claims 1 or 5, **characterised in that** the junction (6) is coated with a heat-conducting and gas-impermeable layer.

7. The device (1) according to one of the preceding claims, **characterised in that** the device (1) further comprises a second temperature sensor arranged on the temperature control element for determining the temperature of the temperature control element (4).

8. The device (1) according to one of the preceding claims, **characterised in that** the device (1) further comprises a pressure sensor for determining the pressure in the process chamber (2).

9. The device (1) according to one of the preceding claims, **characterised in that** the device (1) further comprises a control and evaluation unit which is designed to determine the dew point temperature on the basis of discontinuities in the temperature curve of the temperature determined by the temperature sensor (4).

10. A heat treatment device (11), in particular sterilisation device comprising a process chamber (2) which can be charged with a process gas, and at least one device (1) for determining the dew point according to one of the preceding claims.

## Revendications

1. Dispositif (1) agencé pour déterminer le point de rosée d'un gaz dans une chambre de traitement (2) comprenant :
- un élément de contrôle de la température (3) et
- un capteur de température (4) pour déterminer la température d'une surface de mesure rosée du capteur de température (3), qui est en liaison active thermoconductrice avec l'élément de contrôle de la température (3),
dans lequel l'élément de contrôle de la température (3) est conçu pour chauffer et refroidir activement le capteur de température (4),
où
le capteur de température est disposé en contact sur l'élément de contrôle de la température (3), et dans lequel le capteur de température (4) est conçu comme un thermocouple, **caractérisé en ce que**
le dispositif (1) est adapté à la rosée de la jonction (6) d'un premier conducteur (7) et d'un second conducteur (8) du thermocouple (4) comme seule surface du dispositif (1) afin de déterminer le point de rosée du gaz dans la chambre de traitement (2).

2. Dispositif (1) selon la revendication 1, **caractérisé en ce que** le capteur de température (4) et l'élément de contrôle de la température (3) sont isolés électriquement l'un de l'autre.

3. Dispositif (1) selon la revendication 1 ou 2, **caractérisé en ce que** l'élément de contrôle de la température (3) est conçu comme un élément Peltier.

4. Dispositif (1) selon l'une des revendications précédentes, **caractérisé en ce que** l'élément de contrôle de la température (3) est muni d'un dissipateur thermique (5).

5. Dispositif (1) selon la revendication 1, **caractérisé en ce qu'**une couche (9) isolante de la chaleur et imperméable aux gaz entoure le thermocouple (4), à l'exception de la jonction (6) des deux conducteurs (7, 8), et de préférence au moins des parties de l'élément de contrôle de la température (4).

6. Dispositif selon l'une des revendications 1 ou 5, **caractérisé en ce que** la jonction (6) est revêtue d'une couche conductrice de chaleur et imperméable aux gaz.

7. Dispositif (1) selon l'une des revendications précédentes, **caractérisé en ce que** l'appareil (1) comprend en outre un deuxième capteur de température disposé sur l'élément de contrôle de la température pour déterminer la température de l'élément de contrôle de la température (4).

8. Dispositif (1) selon l'une des revendications précédentes, **caractérisé en ce que** le dispositif (1) comprend en outre un capteur de pression pour déterminer la pression dans la chambre de traitement (2).

9. Dispositif (1) selon l'une des revendications précédentes, **caractérisé en ce que** le dispositif (1) comprend en outre une unité de commande et d'évaluation qui est conçue pour déterminer la température du point de rosée sur la base de discontinuités dans le profil de température de la température déterminée par le capteur de température (4).

10. Appareil de traitement thermique (11), en particulier appareil de stérilisation comprenant une chambre de traitement (2) pouvant être chargée d'un gaz de traitement, et au moins un dispositif (1) pour déterminer le point de rosée selon l'une des revendications précédentes.
